Europäisches Patentamt

European Patent Office

Office européen des brevets

(11) Publication number: **0 381 612**
**A1**

## EUROPEAN PATENT APPLICATION

(21) Application number: **90630031.4**

(22) Date of filing: **01.02.90**

(51) Int. Cl.5: **A61B 5/00, G01N 21/77,**
**G01N 21/64**

(30) Priority: **02.02.89 IL 89159**

(43) Date of publication of application:
**08.08.90 Bulletin 90/32**

(84) Designated Contracting States:
**AT BE CH DE DK ES FR GB GR IT LI LU NL SE**

(71) Applicant: **O.C.T. OPTICAL CHEMICAL**
**TECHNOLOGIES LIMITED**
**275 Reuven Street, Neve Habaron**
**IL-30900 Zichron Yaakov(IL)**

(72) Inventor: **Kritzman Amnon**
**35 Herzl Street**
**Zichron Yaakov(IL)**
Inventor: **Falkenstein Eliezer**
**33 Trumpeldor Street**
**Zichron Yaakov(IL)**

(74) Representative: **Waxweiler, Jean et al**
**OFFICE DENNEMEYER S.à.r.l. P.O. Box 1502**
**L-1015 Luxembourg(LU)**

(54) **Medical probe.**

(57) A medical probe apparatus including a relatively rigid probe member defining an end portion configured for penetration through body tissue to a selected body site and including apparatus for providing an output indication of one or more chemical properties at the selected body site.

FIG. 1

## FIELD OF THE INVENTION

The present invention relates to a medical probe which may be introduced into the body and by which one or more body properties may be determined.

## RELATION TO PRIOR PATENT APPLICATIONS

In our prior patent applications listed below (excepting Israel Patent Application No. 88967, filed January 16, 1989), we claimed inter alia a sensing device for use in the manufacture of an optic probe apparatus for quantitative determination of the chemical properties of a medium in situ, the device comprising essentially at least one inorganic (e.g. glass or fused silica) optical fiber means, each of which has at an open end thereof an integral or fused-on porous glass tip, having adsorbed on the internal surface at least one fluorescent or other light sensitive substance. Such device had the advantages over comparative prior art devices of having greater sensitivity, being generally smaller and therefore potentially of greater flexibility and applicability, and in particular being useful for monitoring the chemical properties of the human blood stream in vivo, which was not believed to be practical before.

In the invention of our Israel Patent Application No. 88967, which is to be regarded as an improvement in or modification of the invention of commonly assigned U.S. Application Serial No. 257,929, claiming priority from Israel Patent Applications Nos. 84181 & 84182 (filed October 16, 1987), and 84531 & 84533 (filed November 19, 1987), the adsorbed light sensitive substance is replaced by at least one chemically bonded light sensitive substance. The chemical bonding imparts greater stability to the product than the physically adsorbed substance, e.g. although the previous product had minimum leakage of light sensitive material, in the present product, leakage is zero. The product of the invention of application no. 88967 seems also to be more sensitive than the previous product, and this may be due (at least in certain cases) to the fact that in the case of the chemically bound material there is a more substantial difference between the wave-lengths of the incident and emitted light than is the case for the adsorbed material.

While our aforementioned prior patent applications relate to optic probes, it will be appreciated that the present invention is not restricted thereto.

## BACKGROUND OF THE INVENTION

In surgery (including obstetrics) and medicine generally, it would frequently be desirable to know the state of an internal site in the body prior to undertaking a particular action. For example, in the field of obstetrics, determination of fetal distress immediately prior to birth could be an important factor in making a decision whether or not a caeserian section would be preferable to the normal birth route.

In another field, where amputation is indicated because of a gangrenous condition, it is desirable to know with a reasonable degree of precision, the location of the healthy tissues which should be allowed to remain. These are but two examples (and others will suggest themselves to those skilled in the surgical and medical arts) of instances in which a knowledge of the chemical properties, and in particular, pH and the partial pressures of oxygen and of carbon dioxide, would be extremely useful.

In the instances cited, such properties would inform the skilled person as to fetal distress, and as to the precise location of healthy tissues, respectively. For the purposes indicated, the present invention provides medical probe apparatus, which may utilize, but is not restricted to, optic probes.

Optic probes for quantitative determination of certain chemical properties of the blood in situ, are known. Thus, in US 4476870 (Peterson), which issued October 16, 1984, and the contents of which are incorporated by reference herein, there is described such a probe intended for determining the partial pressure of oxygen in the blood or tissue of a living animal. This probe comprises one or two strands of plastic optical fibers terminating in an elongate section of porous polymer tubing which is packed with a fluorescent visible light-excitable dye placed on a porous adsorptive particulate polymeric support. While many compounds which are excited by ultraviolet light are known, and the intensity of the emitted light of such compounds may be sensitive to the presence of oxygen, Peterson used plastic optical fibers, because (inter alia) he regarded the use of inorganic fibers as impractical for the desired purpose because of brittleness. Also, since plastic optical fibers are insufficiently transparent to ultraviolet light, this necessitated the use by him of visible light and of dyes sensitive thereto.

As will be seen from the description herein, and contrary to the teaching of Peterson, the present inventors have found the use of at least certain inorganic optical fibers to be eminently practical for the purposes of practicing the present invention, and brittleness is not a problem. Moreover, the use of a sensitized porous glass tip at the end of such fibers, enables the manufacture of optical probe apparatus which is believed to be considerably more sensitive than that of Peterson. Furthermore, the use according to the present invention of such a tip has the result that the sensor can be much smaller than, and is therefore potentially of greater flexibility and applicability than Peterson's probe. Unpublished experiments by the present inventors have shown that in fact no more than an ambit of about 200 microns of Peterson's probe is the realistic sensitive volume.

In US 4568518 (Wolfbeis), which issued February 4, 1986 (the contents of which are incorporated by reference herein), there is described a flexible sensor element comprising a carrier membrane and an immobilized network structure (especially one based on cellulose) including a fluorescent indicator, and in particular such an indicator for measurement of pH values and for blood gas analysis. Wolfbeis's object appears to be to load the carrier with as much indicator as possible and he takes the view that "all known methods of immobilization pertaining to glass surfaces suffer from the disadvantage that the surface will take up only a relatively small amount of bonded immobilized material in a single layer."

Contrary to the teaching of Wolfbeis, however, the present inventors have found that the fact that glass surfaces may only immobilize a relatively small amount of material (in the present invention the relevant material is chemically bonded to internal surfaces of the glass, optimally in a single layer) is to be regarded as an advantage and not a disadvantage. This is because when much more than a molecular layer of fluorescent material is immobilized on a carrier, the fluorescent molecules under excitation may tend to react physically with each other, the effect of which will be to substantially reduce the number of excited molecules which give the desired information.

In European Patent Application No. 0214768 (Hirschfeld), published March 18, 1987, the contents of which are incorporated herein by reference, physical and chemical properties of a sample fluid are monitored by measuring an optical signal generated by a fluorescent substance and modulated by an absorber substance. In practice, both fluorescent and absorber substances may be adsorbed on or/and covalently bonded to glass in the form of porous or sintered glass, or a colored filter glass may be used alternatively as substrate, the substrate in each case being attached by adhesive to one end of a fiber optic. There is no suggestion in this European Patent Application either that the fluorescent substance may be used in absence of the absorber substance, or that the device of the invention may be used for the measurement of chemical properties of the blood in situ, although the same inventor in U.S. Patent No. 4,599,901, which issued July 15, 1986 (and the contents of which are incorporated by reference herein) described a method for direct measurement of arterial blood pressure measuring the intensity of emissions from (inter alia) the surface of a plastic bubble coated with a fluorescent composition and attached to the end of a fiber optic. Moreover, the teaching of the use of porous glass in EP 0214768 is restricted to the use of commercially available material which is attached by adhesive to the fiber optic; any concept of attachment in any other manner, or of formation of the porous glass sensor element in situ is completely absent.

In principle, in the embodiment of the present invention which utilizes an optic probe, the essential elements of the latter are (except for an optional surface polymeric film) virtually completely fabricated from suitable inorganic materials such as glass and/or fused silica without the aid of adhesive for bonding the sensor, and which it is believed, when having chemically bonded to the internal surface thereof one or more light sensitive substances, are more sensitive than comparable prior art sensors. The sensor element is also much smaller than hitherto, and this enables the device to be used in the surgical and/or medical contexts noted herein, which it is believed were not from a practical standpoint possible before the advent of the present invention.

It will be seen infra that in a particular embodiment, the present invention makes use of inter alia substantially nonporous glasses ("parent glasses") which are convertible to porous glasses. Both parent glasses and porous glasses are well known in the art. When parent glasses, which may be certain borosilicate glasses, are heat-treated there results an interconnected separation of phases, one of which may be leached by acid (or in certain cases even by water) to leave an insoluble mainly silica phase (in fact, a porous glass) which could be consolidated by heating into a dense, clear glass known in the trade as "Vycor". Since it is the porous glass which is a desirable carrier for chemically bonding at least one light sensitive substance in accordance with the present invention, any such consolidation step as is used to produce "Vycor" glass is of course omitted herein. Composition ranges for parent glasses, which are to be regarded as illustrative only, are:

(1) $SiO_2$ 55-70, $Na_2O$ 10-0.1, $B_2O_3$ balance to make 100%;

(2) $SiO_2$ 55-70, $K_2O$ 9-0.1, $B_2O_3$ balance to

make 100%;

(3) $Al_2O_3$ 0.1-4, $SiO_2$ [55 minus 1.25 x $Al_2O_3$ content] up to 70, $Na_2O$ 10-0.1 [minus 0.17 x $Al_2O_3$ content], $B_2O_3$ balance to make 100%;

(4) $SiO_2$ 55-75, alkalis 5-15, oxides of Fe, Co, Ni 5-15, $B_2O_3$ 15-30%. Literature references to porous glasses are also included in the above-cited European Patent Application.

In GB 1190583 (Bergman), published May 6, 1970, there is described a gas detector for measuring or monitoring the partial pressure of a gas (in particular, oxygen), containing a matrix support for luminescent material. The matrix, which in practice is used in the form of a thin film or disc, may be made of "porous Vycor-type glass". No details are provided of how such glass is obtained. In his illustrated embodiment, a sintered metal cylinder contains the matrix, an ultraviolet glow lamp, filters and photoelectric cells. The atmosphere to be monitored either diffuses through the wall of the cylinder, or is led through the apparatus by inlet and outlet pipes. This patent is not concerned with the use of fiber optics, with a detector element of such a size that it may constitute or be attached to the tip of a fiber optic, or with monitoring the chemical properties of the human blood stream in vivo.

In Weetall and Filbert, Methods in Enzymology, 1974, 34: 59-72, the disclosure of which is incorporated herein by reference, there is described the formation of chemical bonds between porous glass and silicon-containing organic compounds, to which further organic entities can be bound in turn, the products being useful for affinity chromatography.

In Wolfbeis et al, Mikrochimica Acta [Wien], 1984, I: 152-158, the disclosure of which is incorporated herein by reference, there is described a fluorescence sensor for oxygen in which a thin layer of porous glass incorporating chemically bound light sensitive material is mounted on 2 x 2 platelets of conventional glass, which is set into a wall of flow-through apparatus. Fiber optical techniques are not described or suggested.

In Zhou et al, Anal. Chem. 1988, 60: 2317-2320, the disclosure of which is incorporated herein by reference, there is described a flow-through humidity detector containing an optical glass fiber of which a section has been made porous and impregnated with $CoCl_2$, the absorption characteristics of which are utilized for humidity detection.

It will be appreciated that of the prior art described above, only US 4599901, EP 0214768 and the Anal. Chem. 1988 article describe inorganic optical fiber techniques of any kind for detectors, and that none of the prior art either describes or suggests the advantageous use a fused or integral porous glass tips as disclosed in the commonly assigned patent applications referred to above, and which together with the incorporation of chemically bonded materials constitutes the concept preferably incorporated according to a particular embodiment of the present invention.

## SUMMARY OF THE INVENTION

The present invention provides, in one embodiment, medical probe apparatus including a relatively rigid probe member defining an end portion configured for penetration through body tissue to a selected body site and including apparatus for providing an output indication of one or more chemical properties at the selected body site.

Additionally in accordance with an embodiment of the invention, the apparatus for providing comprises one or more probe tips arranged in association with the end portion of said probe member, and apparatus for transmitting a first signal from a signal source to the at least one probe tip and a second signal from the one or more probe tips to a signal receiver.

Further in accordance with an embodiment of the invention, the relatively rigid probe member is an elongate hollow member and the end portion is open, the apparatus for providing being partially enclosed within the probe member and the one or more probe tips being at least partially exposed at the end portion.

In accordance with a preferred embodiment of the invention, the apparatus for transmitting comprises inorganic optical fiber apparatus having a free end adjacent to the end portion of the relatively rigid probe member, and the first and second signals are radiation signals.

There is provided, in accordance with an alternative embodiment of the invention, medical probe apparatus comprising a relatively rigid probe member defining an end portion configured for penetration through body tissue to a selected body site, and apparatus for providing an output indication of one or more chemical properties at the selected body site comprising one or more probe tips arranged in association with the end portion of the probe member, and inorganic optical fiber apparatus having a free end associated with the one or more probe tips for transmitting a first radiation signal from a signal source to the one or more probe tips and for transmitting a second radiation signal from the one or more probe tips to a signal receiver.

According to an embodiment of the invention, the inorganic optical fiber apparatus comprises one or more inorganic optical fibers and each of one or more probe tips comprises a porous glass tip fused-on to or integrally formed with the free end of the optical fiber apparatus, the porous glass tip being characterized by the fact there is chemically bonded to an internal surface thereof one or more substances selected from the group consisting of fluorescent and other light sensitive substances, and the radiation signals are light signals.

Additionally according to an embodiment of the invention, the one or more chemical properties is selected from the group consisting of pH, partial pressure of oxygen, and partial pressure of carbon dioxide.

Further according to an embodiment of the invention, one or more inorganic optical fiber is selected from the group consisting of glass and fused silica optical fiber apparatus.

## BRIEF DESCRIPTION OF THE DRAWINGS

The present invention will be more fully understood and appreciated from the following detailed description, taken in conjunction with the drawings, in which

Fig. 1 shows in section a sensing device useful according to an embodiment of the invention which comprises a single unbranched inorganic optical fiber;

Fig. 2 shows in section a sensing device useful according to a further embodiment of the invention which comprises a single unbranched inorganic optical fiber;

Fig. 3 shows in section an example of a sensing device useful according to the invention which comprises two inorganic optical fibers which in one embodiment are attached to a single glass tip and in another embodiment are fused together at one end;

Fig. 4 shows in section another sensing device useful according to the invention which comprises two inorganic optical fibers which in one embodiment are attached to a single glass tip and in another embodiment are fused together at one end;

Fig. 5 shows in section a sensing device useful according to an embodiment of the invention which comprises a Y-shaped inorganic optical fiber;

Fig. 6 shows in section a sensing device useful according to a further embodiment of the invention which comprises a Y-shaped inorganic optical fiber;

Fig. 7 shows in section chemical bonding of active material on the inner surface of a single pore of a porous glass tip;

Fig. 8 shows in section a bundle of optical fibers attached to respective glass tips;

Fig. 9 is a block diagram illustration of a probe system constructed in accordance with the present invention;

Fig. 10A shows an end portion of a rigid medical probe, constructed in accordance with an embodiment of the invention;

Fig. 10B shows an end portion of a rigid medical probe, constructed in accordance with an alternative embodiment of the invention; and

Fig. 11 shows a fetal probe constructed in accordance with yet a further embodiment of the invention.

## DETAILED DESCRIPTION OF THE INVENTION

In more detail, the utilized inorganic optical fiber means may be selected e.g. from categories (a), (b) and (c), namely:

(a) glass optical fiber means to each of which is attached by fusion at an open end thereof a discrete glass tip, the glass of the tip being selected from substantially non-porous glasses convertible to porous glasses and wherein the tip has been subjected to an after-treatment to convert it to a porous glass tip;

(b) fused silica optical fiber means to each of which is attached by fusion at an open end thereof a discrete glass tip, the glass of the tip being selected from substantially non-porous glasses convertible to porous glasses and wherein the tip has been subjected to an after-treatment to convert it to a porous glass tip;

(c) glass optical fiber means fabricated from a substantially non-porous glass convertible to a porous glass, an open end of each of which defines the tip thereof and wherein the tip has been subjected to an after-treatment to convert it to a porous glass tip.

The at least one light sensitive substance preferably comprises a substance such that when incident light of a known exciting (first) wave-length impinges thereon, it will emit light of a particular (second) wave length when present in below a preselected concentration per unit internal surface area and it will commence to emit light in a significant and detectable quantity of a particular (third) wave length when present in at least the preselected concentration per unit internal surface area. The substance having the properties just described is desirably present in at least the preselected concentration per unit internal surface area. By way of example, this substance may contain a pyrene ring system.

The sensing device of the invention may be made, e.g., by a process comprising the step of attaching by fusion a glass tip to an open end of each one of at least one inorganic (e.g. glass or fused silica) optical fiber means, and wherein the glass of the tip is selected from substantially non-porous glasses convertible to porous glasses, or alternatively by a process comprising the step of treating an open end at the tip thereof of each one of at least one glass optical fiber means fabricated from a substantially non-porous glass convertible to a porous glass, thereby to convert the or each tip to a porous glass tip.

It is to be understood that the probe of the invention comprises in one embodiment a glass tip either fused to or integral with an open end of inorganic optical fiber means (or a plurality of glass tips fused to or integral with respective open ends of a plurality of inorganic optical fiber means), which when used as the sensor of an optic probe will have been made porous, and will then have chemically bonded to the internal surface thereof at least one light sensitive substance.

A "light sensitive substance" in the present context means a substance which will react to incident light by emitting a light signal. It is presently preferred that this substance is an agent which imparts information exclusively by means of the internally unmodified intensity of the light emitted therefrom. In other words, it is preferred not to add any substance, such as the absorber of EP 0214768, which will vary the intensity of the emitted light signal. The intensity of the emitted signal will of course be modified by an external agent, namely, by the particular chemical property of the medium, the determination of which is desired.

The sensitivity of such substance (which may be a fluorescent substance) to light, e.g. visible or ultraviolet light, will be affected by the chemical properties of the medium in which the sensor is inserted. Among the applications of the present invention, there are included the determination in vivo of the mentioned chemical properties of blood, but use of the invention is not to be construed as limited thereto.

By the expression "at least one inorganic optical fiber means" it is intended to convey that the invention includes, on the one hand, either one or a plurality of inorganic optical fiber means, and on the other hand that what is termed "inorganic optical fiber means" may assume a number of configurations of inorganic optical fibers. Thus, for example, each inorganic optical fiber means may be constituted in one or other of the following modes, namely, where: (i) the inorganic optical fiber means comprises one unbranched inorganic optical fiber; (ii) the inorganic optical fiber means comprises at least two inorganic optical fibers all of which are attached to, or are fused together to form, a single glass tip; (iii) the inorganic optical fiber means is Y-shaped, and the glass tip (which is porous or is to be made porous) is located at an open end of one of the arms of the Y. In mode (i), it is evident that both the light directed to the sensor and that emitted therefrom will be channeled along the same optical fiber. In mode (ii), it is intended that the light directed to the sensor will be channeled along one (or more) optical fibers, and the emitted light will be mainly channeled along another one (or more) optical fibers. In mode (iii), it is intended that the light directed to the sensor will be channeled along one arm of the Y to the junction and thereafter to the sensor (i.e. the sensitized tip), while the emitted light will be channeled from the sensor to the junction and thereafter (mainly) along the other arm of the Y. Whatever the manner in which the or each inorganic optical fiber means is constituted, the device of the invention may comprise one or a plurality of such inorganic optical fiber means.

When the probe of the invention comprises a single inorganic optical fiber means, it will be most readily adapted to the determination of a single chemical property. Nevertheless, it is within the scope of the present invention to chemically bond to the internal surface of a single porous tip, more than one light sensitive substance and to measure the light emitted from each such substance present in the porous tip, in order to determine more than one chemical property. In an alternative embodiment of the probe of the invention, in which the "at least one inorganic optical fiber means" comprises in fact a plurality of inorganic optical fiber means, each of which has attached thereto or integral therewith a different porous inorganic tip, then at least two of the tips may have adsorbed thereon different light sensitive substances, respectively, thereby facilitating the determination of at least two different chemical properties of the medium.

For the probe according to the invention to be usable, it will be necessary for the (at least one) attached or integral tip to be subjected to a process step whereby the substantially non-porous glass of the tip is converted to a porous glass. This step will normally comprise acid treatment, using e.g. hydrochloric acid, the acid treatment being preceded by a heat treatment. The porous tip or tips thus produced may then be reacted so as to bond to the internal surface thereof at least one light sensitive composition, e.g. a fluorescent composition.

It is advantageous, for a number of reasons, for the thus-treated porous discrete glass tip to be overlaid with a porous polymeric film prior to actual use. Such a film would protect the external glass surface thereof from nicks and scratches, and especially if extended somewhat over the adjacent part of the inorganic fiber means which is either integral with it or to which it is attached, would also have a safety function in preventing any light sensitive substance, from contaminating the medium under test. (However, tests have shown that it is extremely unlikely that under conditions of use generally prevailing, any light sensitive substance will become detached from the internal surface of glass to which it has been chemically bonded.) It will be appreciated that in any event the porosity of the polymeric film will necessarily be such as to allow the ingress of the medium or at least the components thereof which are the subject of quantitative determination. A suitable material for the film would appear to be an RTV silicone such as that marketed under the trade name "Dow Silastic Corning 890 (or 891)", which incidentally has been approved by the FDA for body implants. It is also possible to overlay the end of the inorganic optical fiber with a preformed polymeric tube (e.g. that marketed under the trade name "Celgard"), the end of which can be closed and (if necessary) adhered to the outer fiber surface by gluing. It may be noted in passing that such gluing does not interfere with the analytical determination as does glue used in the prior art to bond a glass tip to an optical fiber, since it is effected on the outside of the optical fiber and consequently does not obstruct the light path.

The concept of the present invention resides, in the optic probe embodiment, at least partly in the fact that the probe comprises inorganic fiber optic means having integral therewith (in the case of a glass fiber optic means) or fused thereto a glass tip convertible to a porous glass tip, rendering this tip porous, and thereafter chemically bonding to the internal surface thereof at least one light sensitive material. It will therefore be appreciated that such features as the means for sending the light to the sensor, receiving light emitted therefrom, and calculating the desired properties from the intensity of the emitted light, will in principle be known.

Examples of suitable materials which fluoresce when subjected to ultraviolet light and which may be useful as the light sensitive substances for being chemically bonded to the internal surfaces of the porous glass tips of the probe of the invention, when used for in vivo or in vitro determinations in the blood (or in industrial applications) are with reference to the determination of oxygen, for example, polycyclic aromatic compounds e.g. pyrene compounds.

Materials fluorescing in light other than UV are also operable.

A general method for attaching organic molecules to porous glass is described, for example, in Weetall and Filbert (loc cit). This general method, which may be applied to chemically bonding light sensitive substances to porous glass tips for the purpose of the present invention, consists of first activating the porous glass e.g. by boiling with nitric acid, then silanizing by reacting the porous glass with an organic molecule containing silylalkoxy group (which reacts with the glass surface) at one end and a functional group (which is available for further reaction) at the other end. Silanization may be effected in organic or in aqueous solution. A typical silanization reagent is $(EtO)_3Si(CH_2)_3NH_2$, but the process is evidently not to be construed as restricted to this particular reagent. Where this reagent (or an analogous reagent containing a primary amino group) is utilized, the residual primary amino group attached through a chain to the glass may be derivatized, e.g. by acylation. Thus for present purposes, pyrene which contains an alkanoic side-chain (such as e.g. pyrenebutyric acid) could be reacted with such residual primary amino groups, as indeed was done by Wolfbeis et al (loc cit). Other fluorescing or otherwise light sensitive residues, besides pyrene, are of course within the scope of the method of Weetall and Filbert and are also within the scope of the present invention, for the purpose of creating chemical bonds between the light sensitive molecules and the internal surfaces of the porous glass tips.

## DETAILED DESCRIPTION OF THE DRAWINGS

It will be appreciated that the drawings are generally schematic and while enlarged compared with the actual size, are not drawn to scale. The same numerals in different drawings depict corresponding features of the illustrated device.

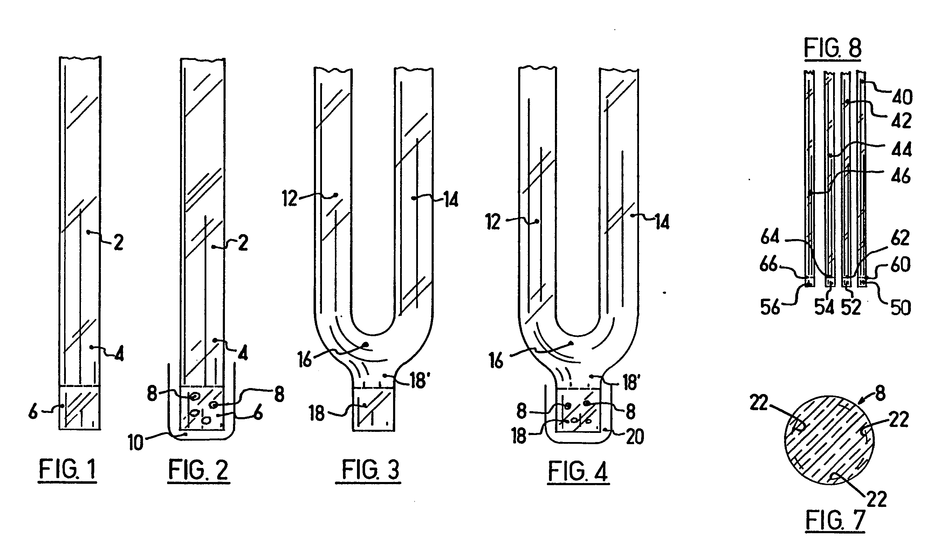

Fig. 1 shows a single unbranched inorganic optical fiber 2, to which is attached at the end 4 thereof, in one embodiment, by fusion, substantially non-porous (but convertible to porous) glass tip 6. In another embodiment, fiber 2 is a single unbranched inorganic optical fiber fabricated from substantially non-porous glass convertible to porous glass, which at the end denoted 4 may potentially be treated as described herein to render the tip porous, e.g. in region denoted 6, up to the broken line. In Fig. 2, tip 6 of the device of Fig. 1 (either embodiment) has been made porous in the manner described herein, and thus now incorporates pores such as those denoted by reference numeral 8. Following adsorption of active substance on the inner surfaces of pores 8, tip 6 as well as at least part of the adjacent portion 4 of glass optical fiber 2 may optionally be coated with porous polymeric film 10.

In Fig. 3, two inorganic optical fibers 12 and 14 are fused together in the vicinity of one end, at region 16; in one embodiment, this end of the fused-together pair of fibers is attached by fusion in region 18 to a single substantially non-porous glass tip 18, whereas in another embodiment, when the fibers are fabricated from substantially non-porous glass convertible to porous glass, the structure already contains a non-porous tip 18, convertible to a porous tip. In Fig. 4, tip 18 of the device of Fig. 3 (either embodiment) has been made porous in the manner described herein, and thus now incorporates pores such as those denoted by reference numeral 8. Following adsorption of active substance on the inner surfaces of pores 8, tip 18, and at least part of the adjacent portion 18', of the inorganic optical fiber, may optionally be coated with porous polymeric film 20.

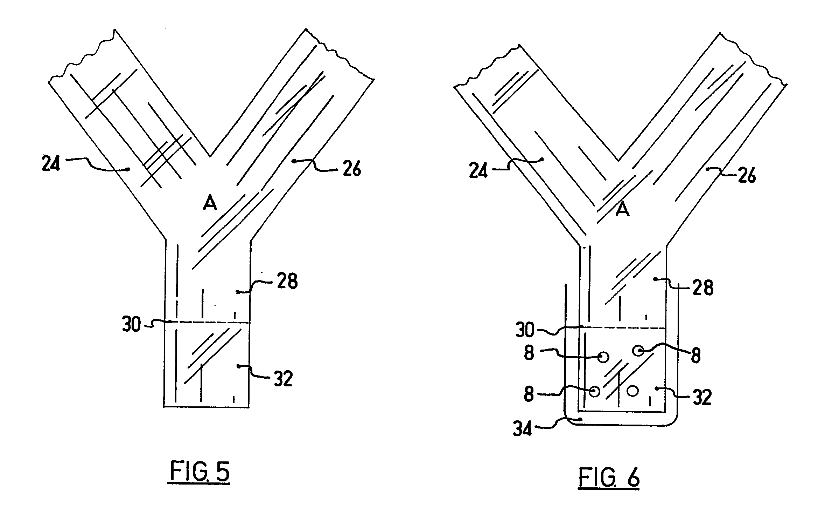

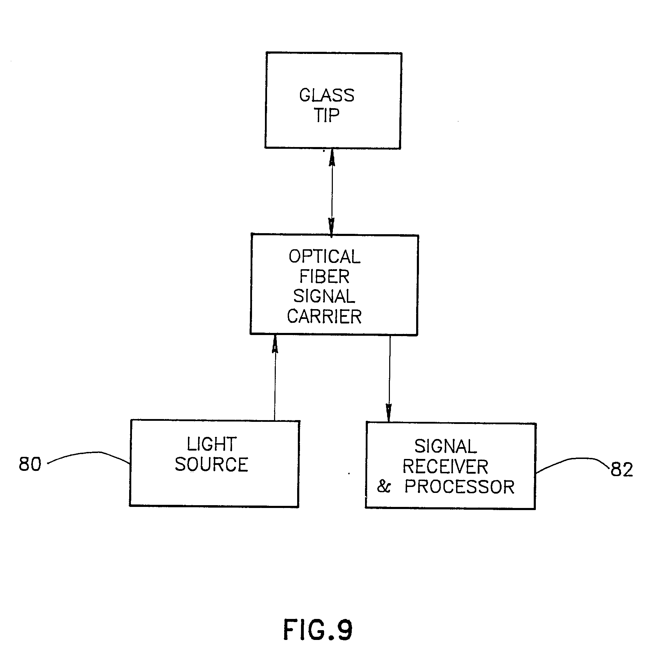

In Fig. 5, a inorganic fiber continuum including Y-shaped junction A from which extends respective arms 24 and 26, is, in one embodiment, attached at end 28 to a substantially non-porous glass tip 32 at fusion region 30; in another embodiment, in which the inorganic fiber continuum is fabricated from substantially non-porous glass convertible to porous glass, 32 represents a region (bounded e.g. by broken line 30) which may potentially be made porous. In Fig. 6, tip 32 of the device of Fig. 5 (either embodiment) has been made porous in the manner described herein, and thus now incorporates pores such as those denoted by reference numeral 8. Following adsorption of active substance on the inner surfaces of pores 8, tip 32 as well as part of the adjacent portion 28 (inclusive of region 30) of the inorganic optical fiber may optionally be coated with porous polymeric film 12. As a variation of the embodiments of Figs. 5 and 6, arm 28 may be replaced by a plurality of separate arms attached (e.g. by fusion) to the apex of the V formed by arms 24 and 26; these separate arms, with respective sensitized tips which are as described either attached thereto or integral therewith, may be used as separate sensors, or as a bundle as described below with reference to Fig. 8.

In the embodiments illustrated in Fig. 2, both the light directed to the sensitized tip 6 and that emitted therefrom will be channeled along the same optical fiber 2. In the embodiments illustrated in Fig. 4, it is intended that the light directed to sensitized tip 6 will be channeled along one of optical fibers 12 and 14, and that the emitted light will be mainly channeled along the other of these optical fibers. In the embodiments illustrated in Fig. 6, it is intended that the light directed to the sensor will be channeled along one arm (24 or 26) of the Y to the junction A and thereafter to the sensitized tip 32, and that the emitted light will be channeled therefrom to junction A and thereafter (mainly) along the other of arms 24 and 26.

Fig. 7 depicts the adsorption of active substance at a number of sites 22 on the inner surface of a typical pore 8.

Fig. 8 shows a bundle of individual inorganic optical fibers 40, 42, 44 and 46. According to one embodiment, to the respective ends of these fibers there are attached by fusion in respective regions 60, 62, 64 and 66, individual tips 50, 52, 54 and 56; according to another embodiment in which the fibers are fabricated from substantially non-porous glass convertible to porous glass, regions 50, 52, 54 and 56, bounded respectively by (e.g.) lines 60, 62, 64 and 66, may be made porous and thereafter sensitized as described herein. The bundle of fibers may in either embodiment be held together by insertion in a flexible sleeve (not shown). In the first mentioned embodiment, the fusion is effected individually with non-porous glass tips convertible to porous glass tips, and the steps of making the tips porous, adsorption of sensitive material and (if desired) coating with a porous polymeric material, are effected as described herein. The optional coating step may of course also be carried out in the case of the second mentioned embodiment. It will be appreciated that in this bundle of fibers, each tip which has been made porous may be sensitized by adsorption of a different substance, thereby making possible the simultaneous analysis of different properties of the medium in which the sensor is inserted. While a bundle of four sensors is shown in Fig. 8, alternatively a bundle of two, three or of more than four sensors, may of course also be utilized.

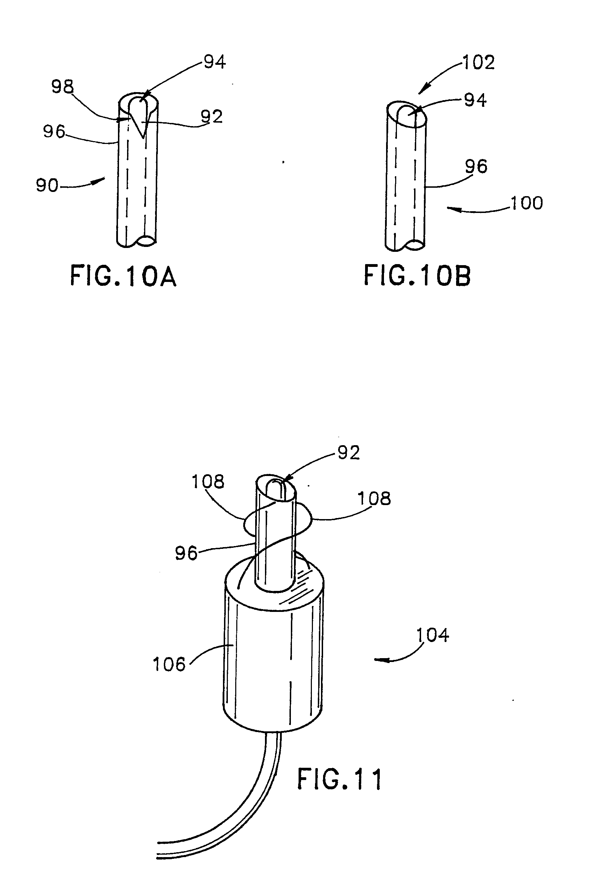

Reference is now made to Fig. 9, which is a block diagram illustration of a probe system constructed in accordance with the present invention. It will be appreciated that the probe system shown may utilize any of the apparatus shown and described above in conjunction with Figs. 1-8 and as shown and described below in conjunction with Figs. 10A-11.

Once the light sensitized glass tip of a sensor of the present invention has been placed in a selected body location whereat it is sought to determine one or more of the properties of pH, partial pressure of oxygen and partial pressure of carbon dioxide, a light source 80 is provided in association with an optical fiber of the sensor. The optical fiber carries an instant, first light signal to the glass tip of the sensor and, a second light signal, as described herein, is emitted therefrom.

The second light signal is carried by the optical fiber from the glass tip to signal receiving and processing apparatus 82, so as to permit evaluation of the reflected signal and determination of the sought chemical property. Light source 80 and signal receiving and processing apparatus 82 may be any suitable apparatus as known to one skilled in the art.

Reference is now made to Fig. 10A, in which there is shown an end portion of a rigid medical probe, referenced generally 90, constructed in accordance with an embodiment of the invention.

Probe 90 typically comprises optical fiber apparatus 92, as shown and described above in accordance with any of Figs. 1-8, having a light sensitive tip portion 94, also according to any of the embodiments of Figs. 1-8.

Fiber apparatus 92 is typically no more than about 140-150 microns in diameter and is supported in a relatively rigid, elongate, outer casing 96. According to the present embodiment, in which probe 90 has a generally needlelike configuration, it may be inserted through body tissue so as to enable determination of properties thereof at a selected location within the body, at a portion of body tissue or within a body cavity. The term 'body tissue' is intended to include blood and non-blood tissue, and bone.

According to a preferred embodiment of the invention, the particular body properties to be determined are one or more of the group including pH, partial pressure of oxygen and partial pressure of carbon dioxide. Furthermore, the use of probe 90 is intended to be for human and nonhuman animals.

Outer casing 96 has a shaped end portion 98 which, according to the shown embodiment, defines a V-shaped notch so as to permit tip portion 94 to be brought into operative association with the body tissue at a selected location, while still providing general mechanical protection to tip portion 94. Outer casing 96 is preferably made of a surgical grade stainless steel and, according to one embodiment of the invention, is a modified surgical needle.

Referring now briefly to Fig. 10B, there is shown an end portion of a rigid medical probe, referenced generally 100, which is similar to probe 90 (Fig. 10A) except that it has a shaped end portion 102 having a truncated configuration. The shaped end portion 102 of probe 100 is also intended to permit operative association of tip portion 94 with the body tissue at a selected location, while still providing general mechanical protection to tip portion 94.

It will be appreciated by persons skilled in the art that the alternative configurations of the end portion of the rigid probe shown in Figs. 10A and 10B are exemplary only, and that many variations of the configurations of the end portion of the probe are envisaged, wherein, for example, the end portion defines a number of notches or serrations.

An application of the rigid probe of either of Figs. 10A or 10B may be in surgery, wherein it would be desirable to know the state of an internal site in the body prior to undertaking a particular action. For example, where amputation of a limb is required because of a gangrenous condition, it is desirable to known with a reasonable degree of precision, the location of the healthy tissues which should be allowed to remain.

In such a case, the rigid probe could be inserted at different locations in the limb and the information thus received would permit relatively accurate determination of the areas of the healthy and unhealthy tissues.

Referring now to Fig. 11, there is shown a fetal probe, referenced generally 104, constructed in accordance with yet a further alternative embodiment of the invention. Probe 104 comprises optical fiber apparatus 92 (Figs. 10A and 10B) located within outer casing 96 (also Figs. 10A and 10B). Outer casing 96 is mounted within a support base 106 to which are also attached hooks 108.

According to the present embodiment, and as is known with fetal ECG monitoring techniques, probe 104 is introduced into the uterus by means of a catheter (not shown), and is adapted to be attached, preferably to selected regions of the skull or the buttocks of the fetus, by twisting it so that the hooks become fastened to the skin of the fetus and such that the tip portion 94 of the optical fiber apparatus 92, becomes operatively engaged with the skin tissue of the fetus.

In this embodiment, therefore, fetal probe 104 may be used to determine fetal characteristics that are known to be indicative of fetal distress immediately prior to birth. Indication of fetal distress could be an important factor in making a decision whether or not a caesarian section would be preferable to the normal birth route.

The particular applications described above in conjunction with Figs. 10A-11 are intended merely as illustrative examples of applications of the described rigid probe, and other instances which a knowledge of the chemical properties of body regions, and in particular, pH and the partial pressures of oxygen and of carbon dioxide, would be extremely useful, will suggest themselves to those skilled in the surgical and medical arts.

The invention will now be illustrated by the following non-limiting examples.

## EXAMPLE 1

Preparation of parent glass and optical fibers having porous glass tips.

(i) Milled sand (674 g.), boric acid (457 g.) and sodium carbonate (119 g.) were melted in a 1 l. alumina crucible at 1500-1520°C in a gas furnace for 1.5 hours. The glass (approximately 1 kg.) was cast, broken into pieces and remelted at 1520°C in an electrical furnace for 2 hours. It was then cast into a steel mold, pressed into a plate about 15 mm. thick and placed in a furnace at 400°C for annealing. This plate was cut to rods ~ 15 x 15 x 40 mm. The resultant parent glass had the composition (wt. %): $SiO_2$ 67.4; $B_2O_3$ 25.7; $Na_2O$ 6.9.

(ii) Graded index 100-140 plastic coated glass optical fibers made by Israel Product Research Co. Ltd., Herzlia, Israel were used in 2.5 m. lengths. Approximately 100 cm. length of the plastic coating was removed at one end of each fiber, using 1,2-dichloroethane; the exposed parts were cleaned well in an ultrasonic bath using successively 1,2-dichloroethane (followed by shaking off excess solvent and air drying), water and ethanol, and finally hot air drying to obtain a product at this stage which was free of grease and moisture.
The parent glass from part (i) (approx. 50 g.) was remelted at 950°C in an electrical furnace. At this temperature the glass was sufficiently fluid to draw fibers. The end of a parent glass fiber was fused in a flame to the end of a commercial glass optical fiber (prepared as described in the preceding paragraph), removing almost all the parent glass, leaving only a small fused glass tip. Alternatively, the ends of commercial glass optical fibers were dipped into molten parent glass, and the fibers were then pulled out with their tips coated with parent glass. In the product, the parent glass tip had a diameter in the range 100 - 500 microns.

(iii) The parent glass tip of the product of part (ii) (produced by either alternative) was heated in an electrical furnace for 25 hours at 610°C, then for 75 hours at 530°C, the heating rate being 3-5°C per minute. The heat-treated tip was then subjected to concentrated aqueous sodium hydroxide for 0.5 hour to remove the surface layer, and leached with 3N aqueous HCl for 25 hours at ~50°C. The specific area of the resultant porous glass tip was 120-140 $m^2$/g.

EXAMPLE 2

Chemical bonding of light sensitive material to porous glass tips.

Several single optical fibers 100 cm. in length and having porous glass tips, prepared as described above, were tied around a 50 cm. glass bar with a band of Teflon (Registered Trade Mark). The edge of the bar with the adjacent porous tips was introduced into a 150 ml. flask and tightened around one its necks with Teflon bands. Activation of the porous tips was effected by refluxing with 10% nitric acid for 18 hours, they were then washed with acetone, rinsed with distilled water and dried in vacuum. The bar with attached fibers was immersed in a solution of 3 ml. $(EtO)_3Si(CH_2)_3NH_2$ -

(Aldrich) in 75 ml. toluene (Merck) containing 0.1 ml. distilled water. Silanization was effected by refluxing for 18 hours, after which the bar with attached fibers was left immersed in distilled water to dissolve residues of polymeric byproducts. After 24 hours, the porous tips were washed with acetone and dried at room temperature for several hours under vacuum.

The glass bar with attached fibers was placed in a tube containing 15 mg. pyrenebutyric acid (PBA) (Aldrich) and 250 mg. dicyclohexylcarbodiimide (Merck) in 10 ml. dry toluene (Merck), secured to the neck of the tube with a Teflon band, allowed to stand for 48 hours, rinsed with toluene and acetone, and left for successive periods of 24 hours in toluene, acetone and dimethylacetamide, separately, in order to remove impurities, in particular unbound PBA.

The sensitivity of the material bonded to the porous glass tip towards oxygen was checked by measuring the fluorescent signal in an Applied Photophysics SP 70/80 spectrofluorometer. The other end of the glass fiber was connected to one end of a Litton Polyscientific beam splitter equipped with a Newport fiber optic positioner. The other two ends of the beam splitter were centered in front of the photomultiplier slit entrance of the spectrofluorometer and at the centre of the slit of the exciting light which was focussed with a quartz lens (focal length = 5 cm.). A strong fluorescent signal was observed by exciting the porous glass tip with light at 348 nm. The maximum of the emission spectrum was recorded at 460 nm which indicates that excimers were formed (as compared with 370-400 nm in absence of excimers). No signs of monomers were detected in the emission band. The fluorescence signal was quenched by pure oxygen at atmospheric pressure by a factor of at least 25.

EXAMPLE 3

Coating the sensors with a porous polymeric film.

A solution of 1 g. RTV silicone marketed under the trade name "Dow Corning Silastic 890 (or 891)-" in 150 ml. toluene was prepared (c.f. Wolfbeis et al, Anal. Chem., 1985 57:2556). The product of the preceding example was dipped therein and curing was effected at room temperature over a 48 hour period. The porous polymeric film extended over the sensitized porous tip and about 10 cm. along the adjacent fiber. The device was now ready for determination of the partial pressure of oxygen. In an alternative procedure, the uncoated sensitized fiber is protected with a sheath of Celgard plastic tube which is adhered to the outside of the fiber with any suitable glue, by way of example EPO-TEK 301 (manufactured by Epoxy Technology Inc., Billerica, Massachusetts, U.S.A.), which is also used to close the end of the overlay tube.

EXAMPLE 4

Correlation of light emission with oxygen content of a test gas.

The product of Example 3 was set up with the sensitized tip in a chamber and in such manner that incident light was led through the fiber to the sensor and emitted light was conducted through the fiber from the sensor to a photomultiplier and means for recording the intensity of the emitted light. 100% nitrogen was led through the chamber at a rate of about 300 c.c./min. and the intensity of the light emitted from the sensor was noted. At approximately 10 second intervals, the composition of the feed gas was changed so as to increase its oxygen content initially from 0 to about 6.67% by volume and thereafter in steps of about 6.67% by volume, so that after 2.5 minutes the gas contained 100X oxygen. The intensity of the light emitted from the sensor was noted at each step of increasing the oxygen content. It was found that over the greater part of the composition range there was a substantially linear correlation between the amount of reduction of the intensity of the emitted light (expressed as a fraction of the intensity of the light emitted with nitrogen only), corresponding with an increase in the oxygen content of the feed gas. A similar result is obtained when the sensor is immersed in an aqueous medium, through which the feed gas is bubbled.

EXAMPLE 5

Preparation of parent glass optical fibers and such fibers having integral porous glass tips.

(i) Parent glass (approx. 50 g.) prepared according to the details given in Example 1 part (i) was remelted at 950°C in an electrical furnace. At this temperature the glass was sufficiently fluid to draw fibers.

(ii) A selection was made from fibers formed according to part (i), for use as optical fibers. The tips were heat-treated in an electrical furnace for 25 hours at 610°C, then for 75 hours at 530°C, the heating rate being 3-5°C per minute. After cooling, the tips of the optical fibers were immersed in 3N aqueous HCl for 25 hours at ~50°C. The specific area of the resultant porous tips of the glass fibers was 120-140 m$^2$/g.

The integral porous tips of the product were reactivated and reacted according to the details given in Example 2, above, with similar results. The resulting sensors were coated with a porous polymeric film as described in Example 3. Correlation of light emission with oxygen content of a test gas was carried out as detailed in Example 4, with similar results.

While embodiments of the invention have been particularly described, it will be appreciated by those skilled in the art that many variations and modifications are possible. For example, although the utilization of glass optical fibers has been described above, it will be evident that other compatible inorganic optical fibers may be substituted for these glass optical fibers at least in the embodiment of the invention wherein the glass optical fibers are fused to glass tips which (as described herein) may be converted to porous tips which are subsequently reacted with light sensitive materials and that the obtained sensors employing such other compatible inorganic optical fibers may be regarded as the chemical and/or mechanical equivalents of those which are particularly described herein. Examples of such are compatible fibers are the fused silica optical fibers obtainable from such suppliers as Fiberguide Industries, Polymicro Technologies and Ensign-Bickford Optics Company (U.S.A.). Preliminary experiments by the present inventors have shown that the substitution of such fused silica optical fibers for the glass optical fibers give products which appear to give less background noise and therefore possess potentially even more sensitivity than the glass fiber products. The invention is therefore not to be construed as limited by the embodiments particularly described herein, but the principles thereof may be practised within the spirit of the invention as will be apparent to those skilled in the art.

**Claims**

1. Medical probe apparatus comprising:
a relatively rigid probe member defining an end portion configured for penetration through body tissue to a selected body site and including means for providing an output indication at the selected body site of at least one chemical property, preferably selected from the group consisting of pH, partial pressure of oxygen, and partial pressure of carbon dioxide.

2. Apparatus according to claim 1, and wherein said means for providing comprises:

at least one probe tip arranged in association with said end portion of said probe member; and

means for transmitting a first signal from a signal source to said at least one probe tip and a second signal from said at least one probe tip to a signal receiver, said means for transmitting being preferably inorganic optical fiber means, and said signals being preferably radiation signals.

3. Apparatus according to claim 2, and wherein said relatively rigid probe member is an elongate hollow member which is preferably substantially cylindrical and said end portion is open, said means for providing being at least partially enclosed within said probe member and said at least one probe tip being at least partially exposed at said end portion, and said end portion preferably defining at least one notch so as to ease penetration thereof through body matter.

4. Apparatus according to either claim 2 or claim 3 and comprising additionally a signal source and a signal receiver, and wherein said free end of said inorganic optical fiber means is associated with said at least one probe tip for transmitting a first radiation signal from said signal source to said at least one probe tip and for transmitting a second radiation signal from said at least one probe tip to said signal receiver.

5. Apparatus according to any of claims 2 to 4, wherein said inorganic optical fiber means is preferably selected from glass and fused silica optical fiber means, and comprises at least one inorganic optical fiber and each of said at least one probe tip comprises a porous glass tip either fused onto or integrally formed with said free end of said optical fiber means, said porous glass tip being characterized by the fact there is chemically bonded to an internal surface thereof at least one substance selected from the group consisting of fluorescent and other light sensitive substances, and preferably comprising at least one fluorescent substance, such as one containing a pyrene ring system, which is sensitive to ultraviolet light; and said radiation signals are light signals.

6. Apparatus according to claim 5, and wherein said probe tip is a glass tip and said inorganic optical fiber means is selected from the group consisting of categories (a), (b) and (c), namely:

(a) glass optical fiber means to each of which is attached by fusion at said free end thereof said glass tip, the glass of said tip being selected from the group consisting of substantially nonporous glasses convertible to porous glasses and wherein said tip has been subjected to an aftertreatment to convert it to a porous glass tip;

(b) fused silica optical fiber means to each of which is attached by fusion at said free end thereof said glass tip, the glass of said tip being selected from the group consisting of substantially nonporous glasses convertible to porous glasses and wherein said tip has been subjected to an aftertreatment to convert it to a porous glass tip;

(c) glass optical fiber means fabricated from a substantially nonporous glass convertible to a porous glass, said free end of each of which defines said glass tip and wherein said tip has been subjected to an after-treatment to convert it to a porous glass tip.

7. Apparatus according to claim 6, and wherein said inorganic optical fiber means is constituted in one of the following modes, namely:

it comprises a single inorganic optical fiber means;

it comprises a plurality of inorganic optical fiber means, each of which in the case of categories (a) and (b) has attached thereto a different discrete porous glass tip;

it comprises one unbranched inorganic optical fiber;

it comprises at least two inorganic optical fibers all of which are attached to or are integral with a single porous glass tip;

it is Y-shaped.

8. Apparatus according to any of claims 5 to 7, wherein said optical fiber means comprises a plurality of optical fiber means each of which has attached thereto or integral therewith a different porous glass tip each of which has chemically bonded to the internal surface thereof a substance selected from the group consisting of fluorescent and other light sensitive substances, such that at least two porous glass tips have different respective chemically bonded substances, respectively, whereby said probe apparatus is adapted for the determination of at least two different chemical properties at said selected site.

9. Apparatus according to any of claims 5 to 8, wherein each porous glass tip has an overlay of a porous polymeric film, which preferably extends to a portion of said optical fiber means adjacent to each porous tip.

10. Apparatus according to any of claims 5 to 9, wherein said at least one substance is an agent which imparts information exclusively by means of the internally unmodified intensity of the light emitted therefrom.

11. Apparatus according to any of claims 5 to 10, wherein said at least one substance comprises a substance such that when incident light of a known exciting (first) wavelength impinges thereon, it will emit light of a particular (second) wave length when present in below a preselected concentration per unit internal surface area and it will commence to emit light in a significant and detectable quantity of a particular (third) wave length when present in at least said preselected concentration per unit internal surface area.

12. Apparatus according to any of claims 5 to 11, wherein said chemical bonding has been effected in two stages, the first stage comprising an optional preliminary activation treatment of the porous glass, for example using nitric acid, followed by silanization of the internal surface of the porous glass by reacting the latter with an organosilicon compound containing a reactive first functional group (such as primary amino) and the second stage comprising reacting said first functional group with a preselected light sensitive compound containing a second functional group (such as a carboxylic group or a reactive derivative thereof) which is reactive with said first functional group.

FIG. 1

FIG. 2

FIG. 3

FIG. 4

FIG. 7

FIG. 8

EP 0 381 612 A1

FIG. 5

FIG. 6

EP 0 381 612 A1

FIG.9

FIG.10A

FIG.10B

FIG.11

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| D,P, X | EP-A-312293 (ISRAEL CERAMIC & SILICATE INSTITUTE) * the whole document * | 1-12 | A61B5/00 G01N21/77 G01N21/64 |
| X | EP-A-283289 (C.R.BARD INC.) * page 6, line 7 - page 7, line 9 * * page 7, lines 39 - 62; figures 1-5 * | 1-4 | |
| A | | 5, 6, 8 | |
| A | EP-A-190829 (GOULD INC.) * abstract; figures 1-3 * | 1, 9 | |
| A | EP-A-190830 (GOULD INC.) * abstract * * page 9, lines 9 - 25; figures 1, 2 * | 1, 5 | |
| A | IEEE TRANSACTIONS ON BIO-MEDICAL ENGINEERING. vol. 33, no. 2, February 1986, NEW YORK US pages 117 - 131; J.L.Gehrich et al.: "Optical Fluorescence and Its Application to an Intravascular Blood Gas Monitoring System" * abstract; figure 2 * | 1-4, 11 | TECHNICAL FIELDS SEARCHED (Int. Cl.5) |
| A | WO-A-8601991 (G.J.LIESE ET AL.) * abstract; figures 1-3 * | 1 | A61B G01N |

The present search report has been drawn up for all claims

| Place of search | Date of completion of the search | Examiner |
|---|---|---|
| THE HAGUE | 10 MAY 1990 | HUNT B.W. |